# EUROPEAN PATENT SPECIFICATION

(11) **EP 0 521 393 B1**
(45) Date of publication and mention of the grant of the patent: **20.03.1996**
(21) Application number: 92110721.5
(22) Date of filing: 25.06.1992
(51) Int. Cl.: C07C 229/42, A61K 31/195, C07C 215/08

(54) **Diclofenac choline salt, a method for the preparation thereof and pharmaceutical compositions containing it**
Diclofenac-Cholinsalz, Verfahren zur Herstellung und pharmazeutische Zusammensetzungen, die es enthalten
Choline sel du diclofénac, procédé pour sa préparation et compositions pharmaceutiques le contenant

(30) Priority: 04.07.1991 IT MI911853
(43) Date of publication of application: 07.01.1993
(73) Proprietor: FARMAKA S.r.l., I-20123 Milano (IT)
(72) Inventor: Di Schiena, Michele Giuseppe, I-20080 Cisliano (Milano) (IT)
(74) Representative: Minoja, Fabrizio

(56) References cited:
- WO-A-82/02889
- BE-A- 887 671
- CH-A- 651 821
- DE-A- 2 935 776
- US-A- 3 558 690

## Description

The present invention relates to diclofenac choline salt, i.e. (2-hydroxyethyl)trimethylammonium [o-(2,6-dichloroanilino)phenyl]acetate, of formula (I) 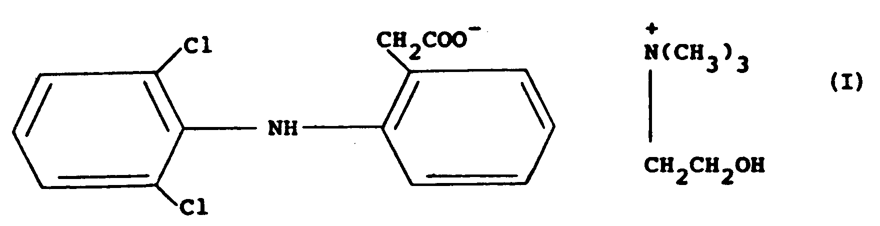
to a process for the preparation thereof, to pharmaceutical compositions containing it and to the use thereof in human and animal therapy as an antiinflammatory, analgesic and antipyretic agent.

Diclofenac is a known antiinflammatory, analgesic and antipyretic drug of the class of phenylacetic acid derivatives. The presence of an amino group at the ortho position to the carboxymethyl group involves, in acid media, the cyclization to lactame with formation of the corresponding indolinone, which is pharmacologically inactive.

US-Pat. 3,558,690, which discloses diclofenac and the preparation thereof, suggests the use of diclorofenac salts with organic and inorganic bases, to stabilize the open form, i.e. the phenylacetic acid structure.

Said salts show a poor water solubility, which makes them unsuitable for the liquid pharmaceutical forms.

As an example, water solubility of sodium diclofenac, which is generally the most used in therapy, is only of 1.36% w/v; and the pyrrolidine and 2-aminoethanol salts are substantially insoluble.

Italian pat. 1,195,318 discloses diclofenac diethylammonium salt which, even though it is very soluble in water, can be used in therapy only in topical preparations, since diethylamine is toxic by the systemic route.

Diethylamine, moreover, can cause nitrosoamines to form, which compounds are known to have a cancerogenic action.

A further drawback of diclofenac is the gastrolesive action, which forbids the oral use thereof in patients suffering from gastro-enteric mucosa impairments, as well as in children and elderly persons; moreover diclofenac has a strong unpleasant taste which is difficult to mask and makes it unacceptable to the oral administration.

One of the more severe side-effects of diclofenac is the hepatotoxic action, therefore it must be carefully used in case of patients suffering from impaired hepatic function, in children and in the elderly persons.

Now it has been found, and it is the object of the present invention, that diclofenac choline salt, which will hereinafter be named diclofenac choline, overcomes the drawbacks mentioned above. In fact, the compound of the invention has surprising chemico-physical and pharmacological properties, such as high water-solubility, up to 25% w/v at room temperature, physiologically compatible pH and stability of the aqueous solutions with time, a long-lasting conservation, minor gastrolesive effects, a decreased hepatic toxicity, a better tolerability of the injectable forms, a better taste, the absence of the sodium ion, which allows to use the salt also in nephropathic, cardiopathic and hypertensive patients.

Moreover choline, being a physiological compound which has already been used in therapy, does not suffer from drawbacks related to systemic or local toxicities.

Thanks to such surprising characteristics, the salt of present invention is particularly suitable for the oral, parenteral, rectal, topical administrations, both in human and veterinary therapies.

Diclofenac choline salt can be advantageously be used in various therapeutical fields, such as in internal medicine, traumatology, phlebology, odontostomatology, laryngology, otology, rhinology, ophthalmology, urology, gynecology, proctology, dermatology and the like.

The salt of the invention can be administered in form of pharmaceutical conventional formulations. The compound of the invention is particularly suitable for those formulations which require a high water-solubility of diclofenac, such as preparations in drops, syrups, granulates for the extemporary use, retard forms, effervescent forms, gels for the topical use, vaginal and urethral lavages, injectable preparations, lotions, sprays and the like.

Particularly suitable is the use of the compound of the invention in the formulation of transdermic plasters.

Also the oral forms, suc as capsules, tablets and pills, optionally in gastro-protected form, and the like, are particularly advantageous, due the lower gastrolesivity.

Particularly preferred are the pharmaceutical compositions for the treatment of the oral cavity, such as collutories, gingival gels, medicated tooth-pastes.

The compound of the invention can be administered at doses substantially similar to those of sodium diclofenac.

The oral dose can be for example of 20-200 mg/die; the dose parenteral can vary from 50 to 300 mg/die; the rectal dose can vary from 100 to 400 mg/die; for the topical route, the dose depends on the type of formulation used, the area to treat and the pathology to cure.

For example, in case of a collutory, the dose can be of about 10 mg for each administration; in case of a topical gel, it can be of about 100 mg for each administration.

The compound of the invention can be prepared according to the known methods for the preparation of salts.

For example, diclofenac in the acid form is reacted in equimolar ratios with choline hydroxide in water or in a suitable organic solvent, for example methanol; by subsequent evaporation of the solvent the new salt is obtained.

In a particularly preferred aspect of the invention, choline hydroxide is reacted in water with an excess of acid diclofenac, completely salifying choline. After that, water is evaporated under reduced pressure, the residue is treated with acetone, to obtain the precipitation of the desired compound in high purity, whereas the diclofenac excess and the colored choline degradation products remain in solution, then they are completely removed.

The invention is further illustrated by following examples.

### EXAMPLE 1

### Preparation of choline diclofenac

32.46 g (0.11 mole) of acid diclofenac are added in portions, under stirring, to an aqueous solution containing 12.1 g (0.10 mole) of choline hydroxide.

The mixture is heated to 50°C and left at this temperature for 6-8 hours, checking the absence of the indolinone derivative by means of TLC. Solvent is evaporated off under reduced pressure to a pasty consistence. The residue is taken up into 300 ml of acetone and left under stirring at 0°C overnight, so as to promote and complete the salt crystallization. The crystalline solid is filtered under vacuum and washed with acetone to colorless washing; then it is dried under vacuum or under ventilation at 40°C, to obtain 38 g of choline diclofenac as a white, or nearly white, crystalline solid. M.p.: 178.8-179.7°C (DSC), NMR: in agreement with the assigned structure.

### EXAMPLE 2

### Collutory

100 g contain: diclofenac choline 0.100 g - Sorbidex^{R} 70% 50 g - sodium benzoate 1 g - acesulfame 0.5 g - natural flavor 0.350 g - cochineal dye q.s. - sterile water q.s. to 100 g. Posology: 1 spoonful (about 10 ml - 0.010 g diclofenac choline) for oral washings and gargles, more times a day.

Indications: gingivitis, glossitis, stomatitis, aphthas, paradentitis, paradentosis; side treatment in the conservative and extractive dental therapies; otorhinolaryngologic affections; anginas, pharyngitis, laryngitis, amygdalitis; as a coadjuvant in the post-operative treatments (tonsillectomy, mandible fracture, cyst of the os maxillare, salivary calculosis, mucousitis of the oral cavity caused by chemotherapy, radiotherapy and other physical causes, such as intubation).

### EXAMPLE 3

### Gingival gel

100 g contain: diclofenac choline 0.200 g - CMC AVXF 3 g - glycerin FU 5 g - acesulfame 0.5 g - natural flavor 0.350 g - sterile water q.s. to 100 g - propyl p-oxybenzoate 0.035 g - methyl p-oxybenzoate 0.070 g.

Posology: delicately massage the gingiva with 1/2 - 1 cm of gel 2 or more times a day.

Indications: inflammatory gingivitis, stomatitis, conservative dental therapy, dentin hypersensitivity, side-treatment of pyorrhea alveolaris, teeth extractions, inflammatory conditions caused by prosthesis.

### EXAMPLE 4

### Medicated tooth-paste

Add sodium lauryl sulfate 0.100 g to the formulation of example 3.

Posology: 1/2 - 1 cm of paste, brushing teeth with a smooth toothbrush.

Indications: inflammatory gingivitis, dentin hypersensitivity, side-treatment of pyorrhea alveolaris.

### EXAMPLE 5

### Proctologic gel

100 g contain: diclofenac choline 0.200 g - CMC AVXF 5 g - glycerin FU 5 g - camomile water 10 g - sterile water q.s. to 100 g - propyl p-oxybenzoate 0.035% g - methyl p-oxybenzoate 0.070% g.

Posology: 1/2 - 1 cm of gel 2 or more times a day. In case of internal hemorrhoids, the gel is applied internally through a cannula.

Indications: hemorrhoidal syndrome.

### EXAMPLE 6

### Vaginal lavage

100 g contain: diclofenac choline 0.5 g - povidone 0.300 g - tested perfume q.s. - propyl p-oxybenzoate 0.035 g - methyl p-oxybenzoate 0.070% g - sterile water q.s. to 100 g.

Posology: for vaginal lavages according to the doctor's prescription.

Indications: vulvovaginitis and hexocervicitis of any origins, including those caused by chemo- and radiotherapy; pre- and post-operative prophylaxis in gynecological surgery, in the IUD application, in instrumental surveys; intimate hygiene during puerperium.

### EXAMPLE 7

### Topical gel

100 g contain: diclofenac choline 2.5 g - ethanol 10 g - Idroramnosan^{R} 4 g - glycerin FU 10 g - water-soluble tested perfume q.s. - p-hydroxybenzoic acid esters 0.120 g - depurated water q.s. to 100 g.

Posology: 1 or more applications a day on the interested cutaneous area, delicately massaging until complete absorption.

Indications: painful, phlogistic or traumatic affections of articulations, tendons, ligaments and muscles (arthritis, periarthritis, arthrosynovitis, tendinitis, tenosynovitis, bursitis, contusions, sprains, luxatios, lesions of the meniscus of knee joint, wryneck, lumbago). Phlebitis, periphlebitis, lymphangitis, superficial lymphoadenitis. Erythemas and cutaneous phlogistic processes.

### EXAMPLE 8

### Eye drops

100 g contain: diclofenac choline 2 g - borax 0.800 g - sodium chloride 0.600 g - sodium thiomersal 0.010 g - sterile distilled water q.s. to 100 g.

Posology: instillate into the conjunctival sac 2 or more drops of solution 3-4 times a day, according to the doctor's prescription.

Indications: acute, sub-acute and chronic conjunctivitis; blepharitis and blepharo-conjunctivitis; dacryocystitis, keratitis, scleritis and episcleritis; eye inflammations of post-operative, post-traumatic, heat and chemicals origins.

### EXAMPLE 9

### Ear drops

100 g contain: diclofenac choline 2 g - propylene glycol 60 g - depurated water q.s. to 100 g.

Posology: instillate more drops into the ear 1 or more times a day according to the doctor's prescription.

Indications: in the otologic affections on an inflammatory basis.

### EXAMPLE 10

### Nose gel

100 g contain: diclofenac choline 1.5 g - dequalinium chloride 0.005 g - glycerin FU 5 g - CMC AVXF 5 g - sterile water q.s. to 100 g.

Posology: 2-3 intranasal applications a day.

Indications: affections of nasal mucosa on dermatological basis.

### EXAMPLE 11

### Monodose sachet for extemporary use

One 3 g sachet contains: diclofenac choline 0.030 g - natural flavors 0.150 g - acesulfame 0.020 g - fructose q.s. to 3 g.

Posology: 1 or more sachets a day according to the doctor's prescription, dissolved in half a glass of water.

Indications: in internal medicine, traumatology, phlebology, urology, gynecology as antiinflammatory, analgesic, antipyretic.

### EXAMPLE 12

### Transdermic plaster

Adhesive: synthetic resin
Matrix: acryl polymer
Carrier: propylene glycol
To increase permeability: oleic acid
Protective bandage : silicon polyester film
Support: low density polythene film
Antioxidant in the synthetic resin: butyl hydroxytoluene + butyl hydroxyanisole.

The dose of the active ingredient will depend on the severity of the pathology as well as the patient's conditions and the administration area.

## Claims (Claims for the following Contracting State(s): AT, BE, CH, LI, DE, DK, FR, GB, IT, LU, MC, NL, PT, SE)

1. Compound of formula (I):

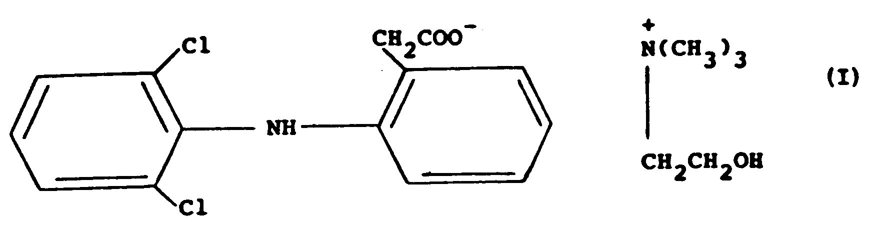

2. A process for the preparation of the compound of claim 1, characterized in that [o-(2,6-dichloroanilino)phenyl]acetic acid is reacted with (2-hydroxyethyl)trimethylammonium hydroxide in water or in methanol and that, after evaporation of said solvent, the resulting salt is crystallized.

3. A process according to claim 2, characterized in that [o-(2,6-dichloroanilino)phenyl]acetic acid is in a molar excess to (2-hydroxyethyl)trimethylammonium hydroxide.

4. A process according to claims 2-3, characterized in that the reaction solvent is water.

5. A process according to claims 3-4, characterized in that the salt is recrystallized from acetone.

6. Pharmaceutical compositions containing the compound of claim 1 as the active ingredient.

7. The use of the compound of claim 1 as a therapeutic agent.

8. The use of the compound of claim 1 for the preparation of a medicament having antiinflammatory, analgesic and antipyretic activities.

## Claims (Claims for the following Contracting State(s): ES, GR)

1. A process for the preparation of a compound of formula (I): 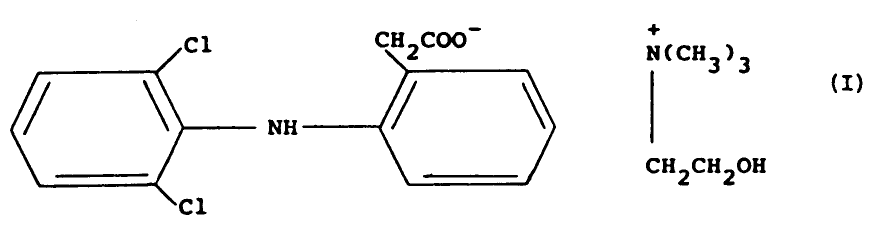 characterized in that [o-(2,6-dichloroanilino)phenyl]acetic acid is reacted with (2-hydroxyethyl)trimethylammonium hydroxide in water or in methanol and that, after evaporation of said solvent, the resulting salt is crystallized.

2. A process according to claim 1, characterized in that [o-(2,6-dichloroanilino)phenyl]acetic acid is in a molar excess to (2-hydroxyethyl)trimethylammonium hydroxide.

3. A process according to claims 1-2, characterized in that the reaction solvent is water.

4. A process according to claims 2-3, characterized in that the salt is recrystallized from acetone.

## Patentansprüche (Patentansprüche für folgende(n) Vertragsstaat(en): AT, BE, CH, LI, DE, DK, FR, GB, IT, LU, MC, NL, PT, SE)

1. Verbindung der Formel (I):

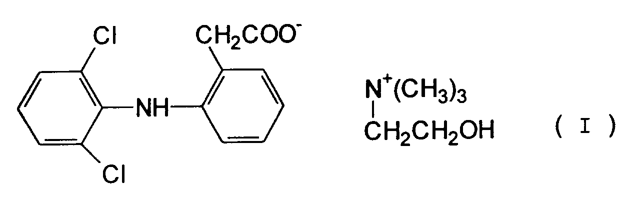

2. Verfahren zur Herstellung der Verbindung nach Anspruch 1, dadurch gekennzeichnet, daß [o-(2,6-Dichloranilino)phenyl]essigsäure mit (2-Hydroxyethyl)trimethylammoniumhydroxid in Wasser oder Methanol umgesetzt wird und daß nach Abdampfen des Lösemittels das entstehende Salz kristallisiert wird.

3. Verfahren gemäß Anspruch 2, dadurch gekennzeichnet, daß [o-(2,6-Dichloranilino)phenyl]essigsäure in einem molaren Überschuß zu (2-Hydroxyethyl)trimethylammoniumhydroxid vorliegt.

4. Verfahren gemäß den Ansprüchen 2-3, dadurch gekennzeichnet, daß das Lösemittel der Reaktion Wasser ist.

5. Verfahren gemäß den Ansprüchen 3-4, dadurch gekennzeichnet, daß das Salz aus Aceton umkristallisiert wird.

6. Pharmazeutische Zusammensetzungen enthaltend die Verbindung gemäß Anspruch 1 als Wirkstoff.

7. Verwendung der Verbindung gemäß Anspruch 1 als therapeutisches Mittel.

8. Verwendung der Verbindung gemäß Anspruch 1 für die Herstellung eines Arzneimittels mit antiinflammatorischen, analgetischen und antipyretischen Wirkungen.

## Patentansprüche (Patentansprüche für folgende(n) Vertragsstaat(en): ES, GR)

1. Verfahren zur Herstellung einer Verbindung der Formel (I): 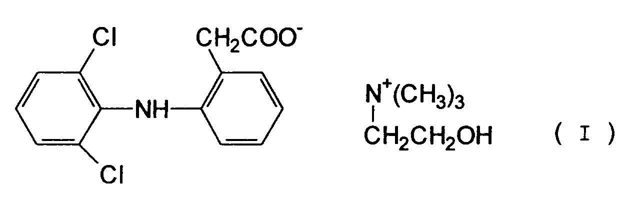 dadurch gekennzeichnet, daß [o-(2,6-Dichloranilino)phenyl]essigsäure mit (2-Hydroxyethyl)trimethylammoniumhydroxid in Wasser oder Methanol umgesetzt wird und daß nach Abdampfen des Lösemittels das entstehende Salz kristallisiert wird.

2. Verfahren gemäß Anspruch 1, dadurch gekennzeichnet, daß [o-(2,6-Dichloranilino)phenyl]essigsäure in einem molaren Überschuß zu (2-Hydroxyethyl)trimethylammoniumhydroxid vorliegt.

3. Verfahren gemäß den Ansprüchen 1-2, dadurch gekennzeichnet, daß das Lösemittel der Reaktion Wasser ist.

4. Verfahren gemäß den Ansprüchen 2-3, dadurch gekennzeichnet, daß das Salz aus Aceton umkristallisiert wird.

## Revendications (Revendications pour l'(les) Etat(s) contractant(s) suivant(s): AT, BE, CH, LI, DE, DK, FR, GB, IT, LU, MC, NL, PT, SE)

1. Composé de formule (I) :

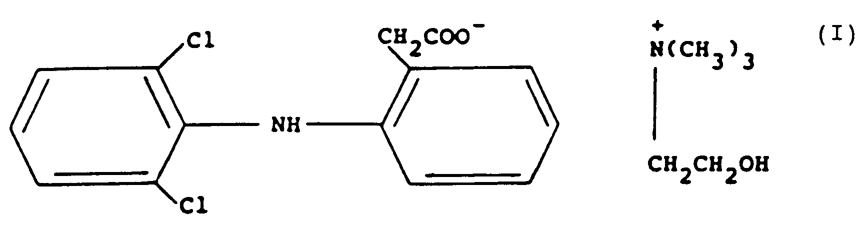

2. Procédé de préparation du composé selon la revendication 1, caractérisé en ce que l'acide [o-(2,6-dichloroanilino)phényl]acétique réagit avec l'hydroxyde (2-hydroxyméthyl)triméthylammonium dans l'eau ou dans le méthanol et que, après évaporation dudit solvant, le sel obtenu est cristallisé.

3. Procédé selon la revendication 2, caractérisé en ce que l'acide [o-(2,6-dichloroanilino)phényl]acétique est en excès molaire par rapport à l'hydroxyde (2-hydroxyéthyl)triméthylammonium.

4. Procédé selon les revendications 2 et 3, caractérisé en ce que le solvant de réaction est l'eau.

5. Procédé selon les revendications 3 et 4, caractérisé en ce que le sel se recristallise à partir de l'acétone.

6. Compositions pharmaceutiques contenant le composé de la revendication 1 en tant qu'ingrédient actif.

7. Utilisation du composé de la revendication 1 en tant qu'agent thérapeutique.

8. Utilisation du composé de la revendication 1 pour la préparation d'un médicament possédant des propriétés antiinflammatoires, analgésiques et antipyrétiques.

## Revendications (Revendications pour l'(les) Etat(s) contractant(s) suivant(s): ES, GR)

1. Procédé de préparation d'un composé de formule (I): 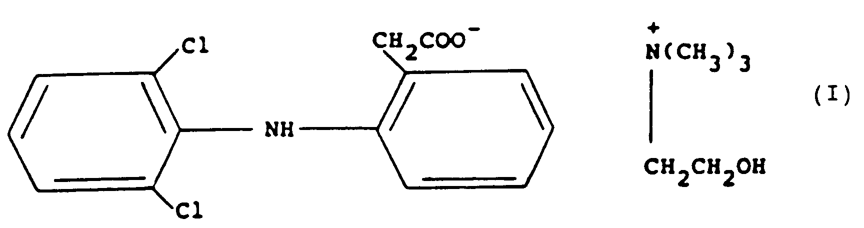 caractérisé en ce que l'acide [o-(2,6-dichloroanilino)phényl]acétique réagit avec l'hydroxyde (2-hydroxyéthyl)triméthylammonium dans l'eau ou dans le méthanol et que, après l'évaporation dudit solvant, le sel obtenu est cristallisé.

2. Procédé selon la revendication 1, caractérisé en ce que l'acide [o-(2,6-dichloroanilino)phényl]acétique est en excès molaire par rapport à l'hydroxyde (2-hydroxyéthyl)triméthylammonium.

3. Procédé selon les revendications 1 et 2, caractérisé en ce que le solvant de réaction est l'eau.

4. Procédé selon les revendications 2 et 3, caractérisé en ce que le sel se recristallise à partir de l'acétone.
